# EUROPEAN PATENT APPLICATION

(11) **EP 1 738 766 A1**
(43) Date of publication of application: **03.01.2007**
(21) Application number: 05727903.6
(22) Date of filing: 30.03.2005
(51) Int. Cl.: A61K 45/00, A61K 31/522, A61P 3/00, A61P 19/02, A61P 19/04, A61P 21/00, A61P 29/00, A61P 43/00, C07D 473/06, C07D 473/12

(54) **PREVENTIVE AND/OR THERAPEUTIC AGENT FOR DISEASE ACCOMPANIED BY CHRONIC MUSCLE/SKELETON PAIN**

(30) Priority: 30.03.2004 JP 2004097422
(71) Applicant: KYOWA HAKKO KOGYO CO., LTD., Chiyoda-ku, Tokyo 100-8185 (JP)
(72) Inventor: KASE, Hiroshi, (JP); TAKAHASHI, Isami, (JP); KUNORI, Shunji, (JP); KOBAYASHI, Minoru, (JP); SHIOZAKI, Shizuo, (JP); SHIRAKURA, Shiro, (JP)
(74) Representative: Vossius & Partner
(86) International application number: PCT/JP2005/006033
(87) International publication number: WO 2005/094885

(57) **Abstract**

A prophylactic and/or therapeutic agent for diseases accompanied by chronic musculoskeletal pain comprising as an active ingredient a compound having an adenosine A_{2A} receptor antagonistic action, for example, represented by the following formula (I) (wherein R¹, R² and R³ are the same or different, each representing a hydrogen atom, lower alkyl, lower alkenyl or lower alkynyl; R⁴ represents cycloalkyl, -(CH₂)ₙ-R⁵ or the following formula (II); and X¹ and X² are the same or different, each representing an oxygen atom or a sulfur atom) or a pharmaceutically acceptable salt thereof.

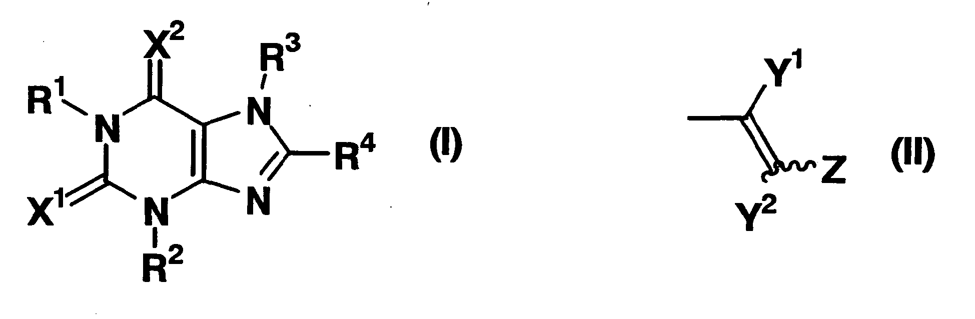

## Description

### Technical Field

The present invention relates to prophylactic and/or therapeutic agents for diseases accompanied by chronic musculoskeletal pain.

### Background Art

Musculoskeletal pain is chronic pain characterized by ache, tenderness, rigidity, and so on, in muscle and skeleton (for example, muscle, tendon, ligament, parts to which the tendon is attached, soft tissues adjacent to them), and as the diseases accompanied by chronic musculoskeletal pain, for example, fibromyalgia syndrome (FMS) and diseases related thereto are known [Merck Index, 17th edition, Chapters 59 and 108; Ann. Pharmacother., 121, p.953-959 (1994)].

Although the cause of onset and pathopysiology of fibromyalgia, which is systemic chronic pain, have not yet been elucidated sufficiently [Expert Opin. Investig. Drugs, 11, p. 1437-1445 (2002)], the lower limit of strength above which sensory stimuli are perceived as pain (pain threshold) is low. Thus, this disease is diagnosed according to the American. College of Rheumatology 1990 Criteria for the Classification of Fibromyalgia, on the basis that the pain spreading a wide range of the body continues over 3 months or longer, and the loci of pain extend to 11 sites or more of the designated 18 sites on the neck and limbs at which the patient will feel pain on pressure [Arthritis Rheum., 33, p.160-172 (1990)]. In addition, this disease is sometimes accompanied by tightening, fatigue, tiredness, exhaustion of physical fitness, sleep disturbance, state of depression, anxiety, autonomic imbalance, headache, irritable bowel syndrome, mild fever, dry eyes, and so on. Objective observation is made based on diagnosis by pushing at the "specific tenderness point" (like a pressure point), but when the whole body is painful, it is sometimes hard to tell which point gives pain on pressure (Merck Index, 17th edition, Chapter 59). In the United States, fibromyalgia is a popular disease of which the number of the patients reaches 2-9% of the population and which occurs mainly in women of 20-50 years of age [Current Opinion in Psychiatry, 13, p.623-628 (2000); Arthritis Rheum., 39, p.19-28 (1990)]. In treatment of these conditions, analgesics, non-steroidal anti-inflammatory agents, muscular relaxants, tricyclic anti-depressants such as amitriptyline, and anti-depressants such as SSRIs (selective serotonin reuptake inhibitors) have been employed. These analgesics and non-steroidal anti-inflammatory agents, however, have no effect in many cases; the muscular relaxants sometimes show a limited effect at a high dose with a remarkable side-effect; the tricyclic anti-depressants or SSRIs show a moderate effect in particular patients but their use is limited due to cardiovascular or anti-cholinergic like side-effect; thus, no effective therapy has been found so far [Expert Opin. Investig. Drugs, 11, p.1437-1445 (2002); Arc. Intern. Med., 156, p.1047-1052 (1996); Ann. Pharmacother., 36, p.707-712 (2002); Arth. Rhem., 33, p.1132 (1990)].

It is known that diseases relating to fibromyalgia syndrome include, for example, fibrositis, chronic fatigue syndrome (CFS), myofascial pain syndrome (MFPS), diffuse myofascial pain syndrome, generalized fibrisitis, soft tissue rheumatism, non-articular rheumatism, chronic rheumatoid arthritis, primary fibromyalgia syndrome (PFS), concomitant fibromyalgia syndrome, idiopathic muscle pain syndrome, chronic widespread musculoskeletal pain, lower back pain, Lyme disease accompanied by fibromyalgia syndrome, generalized tendomyopathy, temporomansibular joint disorder (TMJD), and the like. These diseases relating to fibromyalgia syndrome present the same chronic musculoskeletal pain as or similar to that fibromyalgia syndrome, to which the definition and diagnostic criteria have been proposed respectively; these diseases, however, have not yet definitely been distinguished clinically from fibromyalgia syndrome, and in some cases develop together with or relating to fibromyalgia syndrome [Merck Index, 17th edition, Chapters 59 and 108; Ann. Pharmacother., 121, p.953-959 (1994)].

On the other hand, a number of compounds, for example, xanthine derivatives, [1,2,4]triazolo[1,5-c]pyrimidine derivatives, [1,2,4]triazolo[1,5-a]pyrimidine derivatives, and the like have been known to have an adenosine A_{2A} receptor inhibitory action [for example, US 5,484,920; US 5,703,085; WO 92/06976; WO 94/01114; US 5,565,460; WO 98/42711, WO 00/17201; WO 99/43678; WO 99/26627; WO 01/92264; WO 99/35147; WO 00/13682; WO 00/13681; WO 00/69464; WO 01/40230; WO 01/02409; WO 01/02400; EP 1054012; WO 01/62233; WO 01/17999; WO 01/80893; WO 02/14282; WO 01/97786; WO 03/032996; WO 03/048163; WO 03/049164; WO 03/049165, etc.]. It has not yet been known, however, that the compounds having an adenosine A_{2A} receptor inhibitory action are effective in prophylaxis and/or therapy of diseases accompanied by chronic musculoskeletal pain, for example, fibromyalgia syndrome (FMS), its related diseases, and so on.

### Disclosure of Invention

### Problem to be Solved by the Invention

The object of the present invention is to provide a prophylactic and/or therapeutic agent for diseases accompanied by chronic musculoskeletal pain which comprises as an active ingredient a compound having an adenosine A_{2A} receptor antagonistic action or a pharmaceutically acceptable salt thereof.

### Mean for Solving the Problems

The present invention relates to the following items (1) to (37).
(1) A prophylactic and/or therapeutic agent for diseases accompanied by chronic musculoskeletal pain which comprises as an active ingredient a compound having an adenosine A_{2A} receptor antagonistic action or a pharmaceutically acceptable salt thereof.
(2) The prophylactic and/or therapeutic agent for diseases accompanied by chronic musculoskeletal pain as described in the above item (1), wherein the compound having an adenosine A_{2A} receptor antagonistic action is a xanthine derivative.
(3) The prophylactic and/or therapeutic agent for diseases accompanied by chronic musculoskeletal pain as described in the above item (1), wherein the compound having an adenosine A_{2A} receptor antagonistic action is a xanthine derivative represented by formula (I): 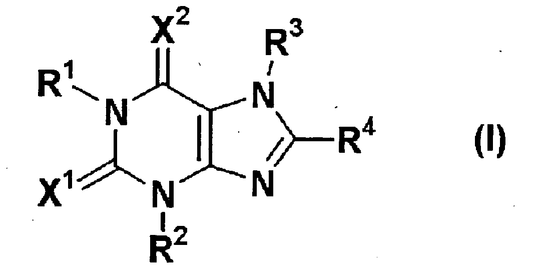 (wherein R¹, R² and R³ are the same or different, each representing a hydrogen atom, lower alkyl, lower alkenyl or lower alkynyl;
   R⁴ represents cycloalkyl, -(CH₂)ₙ-R⁵ (in which R⁵ represents substituted or unsubstituted aryl or a substituted or unsubstituted heterocyclic group; and n is an integer of 0 to 4) or formula (II): 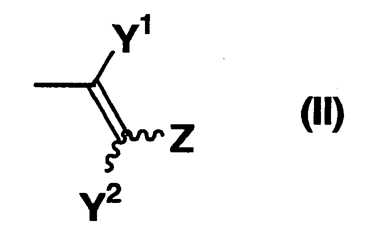 (wherein Y¹ and Y² are the same or different, each representing a hydrogen atom, halogen or lower alkyl; and Z represents substituted or unsubstituted aryl or a substituted or unsubstituted heterocyclic group); and X¹ and X² are the same or different, each representing an oxygen atom or a sulfur atom).
(4) The prophylactic and/or therapeutic agent for diseases accompanied by chronic musculoskeletal pain as described in the above item (3), wherein X¹ and X² each is an oxygen atom.
(5) The prophylactic and/or therapeutic agent for diseases accompanied by chronic musculoskeletal pain as described in the above item (3) or (4), wherein R⁴ is a formula (II): 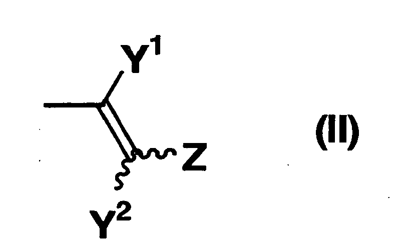 (wherein Y¹, Y² and Z each has the same meanings as mentioned above).
(6) The prophylactic and/or therapeutic agent for diseases accompanied by chronic musculoskeletal pain as described in the above item (5), wherein Y¹ and Y² each is a hydrogen atom.
(7) The prophylactic and/or therapeutic agent for diseases accompanied by chronic musculoskeletal pain as described in the above item (5) or (6), wherein Z is substituted or unsubstituted aryl or a group represented by formula (III): 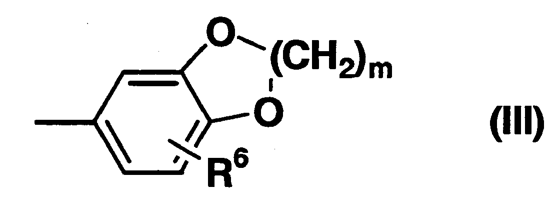 (wherein R⁶ represents a hydrogen atom, hydroxy, lower alkyl, lower alkoxy, halogen, nitro or amino; and m represents an integer of 1 to 3).
(8) The prophylactic and/or therapeutic agent for diseases accompanied by chronic musculoskeletal pain as described in the above item (5) or (6), wherein Z is a group represented by formula (IV): 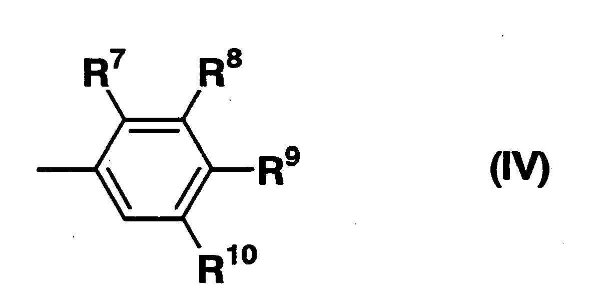 (wherein at least one of R⁷, R⁸ and R⁹ represents lower alkyl or lower alkoxy and the remaining groups represent a hydrogen atom; R¹⁰ represents a hydrogen atom or lower alkyl) or formula (III): 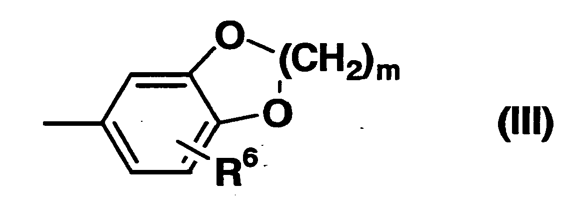 (wherein R⁶ and m each has the same meanings as mentioned above).
(9) The prophylactic and/or therapeutic agent for diseases accompanied by chronic musculoskeletal pain as described in any of the above items (3) to (8), wherein R¹ and R² each is ethyl.
(10) The prophylactic and/or therapeutic agent for diseases accompanied by chronic musculoskeletal pain as described in any of the above items (3) to (9), wherein R³ is methyl.
(11) The prophylactic and/or therapeutic agent for diseases accompanied by chronic musculoskeletal pain as described in the above item (1), wherein the compound having an adenosine A_{2A} receptor antagonistic action is a compound represented by formula (1): 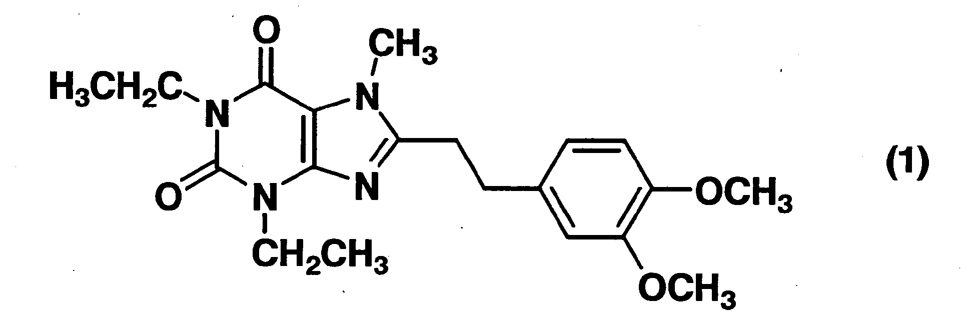
(12) The prophylactic and/or therapeutic agent for diseases accompanied by chronic musculoskeletal pain as described in the above item (1), wherein the compound having an adenosine A_{2A} receptor antagonistic action is a compound represented by formula (V): 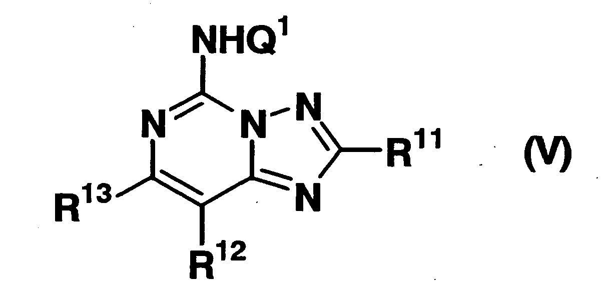 [wherein R¹¹ represents substituted or unsubstituted aryl or a substituted or unsubstituted heterocyclic group;
   R¹² represents a hydrogen atom, halogen, substituted or unsubstituted lower alkyl, substituted or unsubstituted aryl or a substituted or unsubstituted heterocyclic group;
   R¹³ represents a hydrogen atom, halogen or -WR¹⁴ (wherein W represents -O- or -S-, and R¹⁴ represents substituted or unsubstituted lower alkyl, substituted or unsubstituted aryl or a substituted or unsubstituted heterocyclic group);
   Q¹ represents a hydrogen atom or 3,4-dimethoxybenzyl] (for example, 5-amino-7-(4-benzopiperazinyl)-2-,(2-furyl)[1,2,4] triazolo[1,5-c]pyrimidine, and the like).
(13) The prophylactic and/or therapeutic agent for diseases accompanied by chronic musculoskeletal pain as described in the above item (1), wherein the compound having an adenosine A_{2A} receptor antagonistic action is a compound represented by formula (VI): 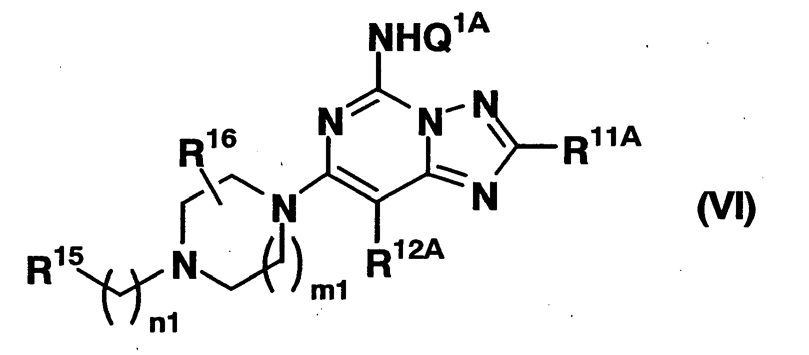 [wherein R^{11A} represents substituted or unsubstituted aryl or a substituted or unsubstituted aromatic heterocyclic group; R^{12A} represents a hydrogen atom, halogen, substituted or unsubstituted lower alkyl, substituted or unsubstituted aryl or a substituted or unsubstituted aromatic heterocyclic group;
   n1 and m1 are the same or different, each representing an integer of 0 to 4;
   Q^{1A} represents a hydrogen atom or 3,4-dimethoxybenzyl;
   R¹⁵ represents a hydrogen atom, substituted or unsubstituted aryl, a substituted or unsubstituted heterocyclic group or -CR¹⁷R¹⁸R¹⁹ (wherein R¹⁷, R¹⁸ and R¹⁹ are the same or different, each representing a hydrogen atom, hydroxy, substituted or unsubstituted lower alkyl, substituted or unsubstituted lower alkoxy, substituted or unsubstituted aryl or a substituted or unsubstituted heterocyclic group, or R¹⁸ and R¹⁹ , taken together with the adjacent carbon atom, form a substituted or unsubstituted carbocycle);
   R¹⁶ represents a hydrogen atom, halogen, hydroxy or substituted or unsubstituted lower alkyl] (for example, 5-amino-2-(2-furyl)-7-[4-(2-hydroxy-2-methylpropyl)-piperazinyl][1,2,4]triazolo[1,5-c]pyrimidine, 5-amino-2-(2-furyl)-7-[4-(3-hydroxy-3-methylpropyl)-piperazinyl][1,2,4]triazolo[1,5-c]pyrimidine, and the like) .
(14) The prophylactic and/or therapeutic agent for diseases accompanied by chronic musculoskeletal pain as described in the above item (13), wherein R¹⁵ represents -CR^{17A}R^{18A}R^{19A} (wherein R^{17A} represents hydroxy, hydroxy lower alkyl, substituted or unsubstituted lower alkoxy or imidazo[1,2-a]pyridyl; R^{18A} and R^{19A} are the same or different, each representing a hydrogen atom, substituted or unsubstituted lower alkyl or substituted or unsubstituted aryl, or R^{18A} and R^{19A} , taken together with the adjacent carbon atom, form a substituted or unsubstituted carbocycle).
(15) The prophylactic and/or therapeutic agent for diseases accompanied by chronic musculoskeletal pain as described in the above item (1), wherein the compound having an adenosine A_{2A} receptor antagonistic action is a compound represented by formula (VII): 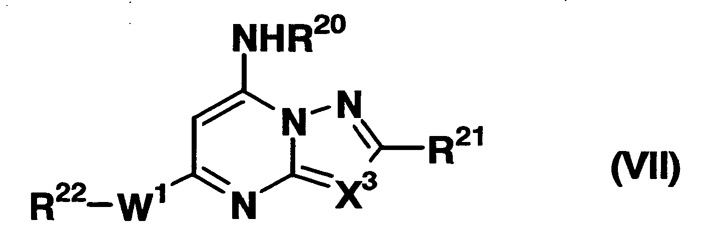 [wherein R²⁰ represents a hydrogen atom, substituted or unsubstituted lower alkyl or substituted or unsubstituted lower alkanoyl;
   R²¹ represents a substituted or unsubstituted heterocyclic group;
   R²² represents a hydrogen atom, substituted or unsubstituted lower alkyl, substituted or unsubstituted lower alkenyl, substituted or unsubstituted cycloalkyl, substituted or unsubstituted aryl or a substituted or unsubstituted heterocyclic group;
   W¹ represents a single bond, -O-, -S-, -S(=O)-, -S(=O)₂- or -NR²³- (wherein R²³ represents a hydrogen atom or substituted or unsubstituted lower alkyl);
   X³ represents a nitrogen atom or CR²⁴ (wherein R²⁴ represents a hydrogen atom or substituted or unsubstituted lower alkyl)].
(16) The prophylactic and/or therapeutic agent for diseases accompanied by chronic musculoskeletal pain as described in the above item (1), wherein the compound having an adenosine A_{2A} receptor antagonistic action is a compound represented by formula (VIII): 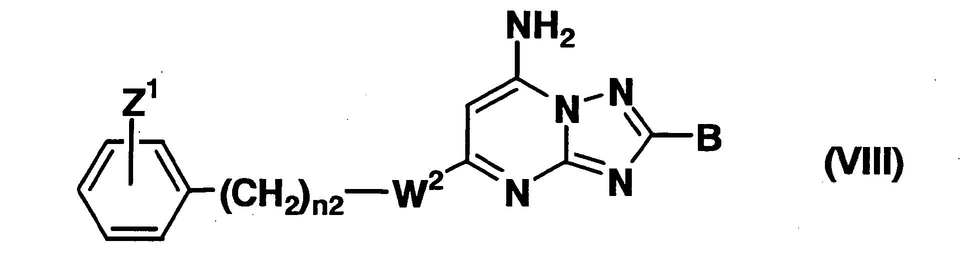 (wherein B represents furyl or thienyl;
   w² represents a single bond, -O- or -S-;
   Z¹ represents a hydrogen atom, halogen or substituted or unsubstituted lower alkyl; and
   n2 represents an integer of 0 to 5).
(17) The prophylactic and/or therapeutic agent for diseases accompanied by chronic musculoskeletal pain as described in the above item (1), wherein the compound having an adenosine A_{2A} receptor antagonistic action is a compound represented by formula (IX): 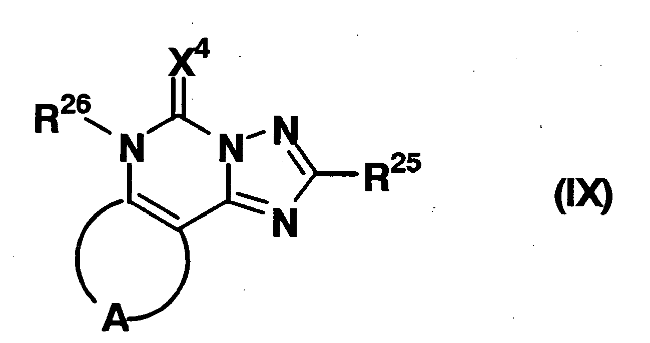 [wherein R²⁵ represents substituted or unsubstituted aryl or a substituted or unsubstituted heterocyclic group;
   R²⁶ represents a hydrogen atom, substituted or unsubstituted lower alkyl, substituted or unsubstituted lower alkenyl, substituted or unsubstituted alkynyl, substituted or unsubstituted cycloalkyl, substituted or unsubstituted aryl or a substituted or unsubstituted heterocyclic group;
   X⁴ represents an oxygen atom, a sulfur atom or NR²⁷ (wherein _{R}²⁷ represents a hydrogen atom, substituted or unsubstituted lower alkyl, substituted or unsubstituted cycloalkyl or substituted or unsubstituted aryl);
   A, taken together with the adjacent two carbon atoms, form a substituted or unsubstituted carbocycle or a substituted or unsubstituted heterocycle].
(18) The prophylactic and/or therapeutic agent for diseases accompanied by chronic musculoskeletal pain as described in the above item (1), wherein the compound having an adenosine A_{2A} receptor antagonistic action is a compound represented by formula (X): 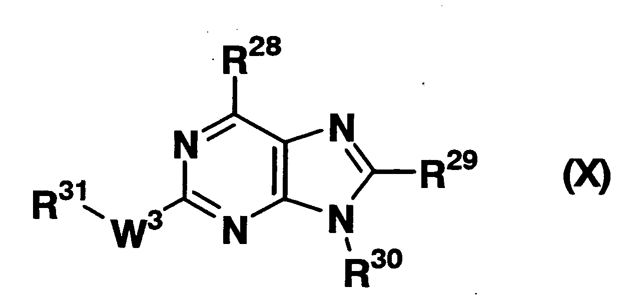 (wherein R²⁸ represents a hydrogen atom, hydroxy, substituted or unsubstituted lower alkyl, substituted or unsubstituted amino or a substituted or unsubstituted heterocyclic group;
   R²⁹ represents a hydrogen atom, substituted or unsubstituted lower alkenyl, substituted or unsubstituted cycloalkyl, substituted or unsubstituted aryl or a substituted or unsubstituted aromatic heterocyclic group;
   R³⁰ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl or a substituted or unsubstituted aromatic heterocyclic group; R³¹ represents substituted or unsubstituted lower alkyl or substituted or unsubstituted aryl; and
   W³ represents -CH₂CH₂-, -CH=CH- or -C≡C-).
(19) The prophylactic and/or therapeutic agent for diseases accompanied by chronic musculoskeletal pain as described in the above item (1), wherein the compound having an adenosine A_{2A} receptor antagonistic action is a compound represented by formula (XI): 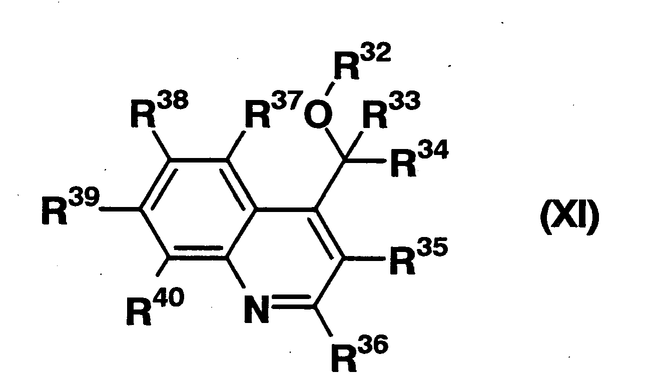 (wherein R³² represents a hydrogen atom or substituted or unsubstituted lower alkyl;
   R³³ and R³⁴ are the same or different, each representing a hydrogen atom, substituted or unsubstituted lower alkyl or substituted or unsubstituted aryl;
   R³⁵, R³⁶, R³⁷, R³⁸ , R³⁹ and R⁴⁰ are the same or different, each representing a hydrogen atom, substituted or unsubstituted lower alkyl, substituted or unsubstituted aryl or a substituted or unsubstituted heterocyclic group).
(20) The prophylactic and/or therapeutic agent for diseases accompanied by chronic musculoskeletal pain as described in the above item (1), wherein the compound having an adenosine A_{2A} receptor antagonistic action is a compound represented by formula (XII): 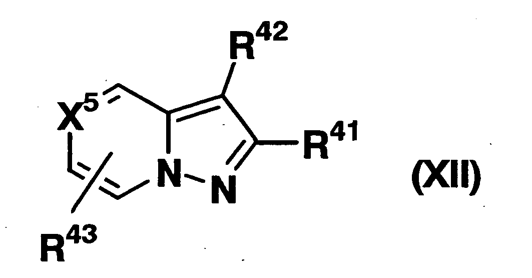 (wherein R⁴¹ represents substituted or unsubstituted lower alkyl, substituted or unsubstituted aryl or a substituted or unsubstituted heterocyclic group;
   R⁴² represents substituted or unsubstituted lower alkyl or a substituted or unsubstituted heterocyclic group;
   R⁴³ represents hydroxy, halogen or substituted or unsubstituted lower alkyl;
   X⁵ represents a nitrogen atom or CH) (for example, 3-[2-(thiazol-2-ylmethyl)-3-oxo-2,3-dihydropyridazin-6-yl]-2-phenylpyrazolo[1,5-a]pyridine and the like).
(21) The prophylactic and/or therapeutic agent for diseases accompanied by chronic musculoskeletal pain as described in the above item (1), wherein the compound having an adenosine A_{2A} receptor antagonistic action is a compound represented by formula (XIII): 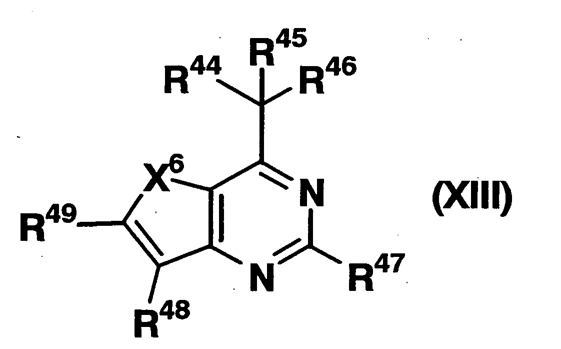 (wherein R⁴⁴ and R⁴⁵ are the same or different, each representing a hydrogen atom, hydroxy, cyano, nitro, substituted or unsubstituted lower alkyl, substituted or unsubstituted lower alkoxy or substituted or unsubstituted aryl, or R⁴⁴ and R⁴⁵, taken together, form oxo, hydroxyimino, imino or hydrazono; R⁴⁶ represents substituted or unsubstituted lower alkyl or substituted or unsubstituted aryl;
   R⁴⁷, R⁴⁸ and R⁴⁹ are the same or different, each representing hydroxy, halogen, cyano, nitro, substituted or unsubstituted lower alkyl, substituted or unsubstituted lower alkoxy or substituted or unsubstituted aryl;
   X⁶ represents an oxygen atom or a sulfur atom).
(22) The prophylactic and/or therapeutic agent for diseases accompanied by chronic musculoskeletal pain as described in the above item (1), wherein the compound having an adenosine A_{2A} receptor antagonistic action is a compound represented by formula (XIV): 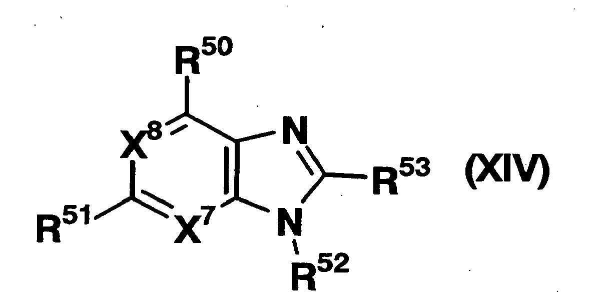 (wherein R⁵⁰ represents a hydrogen atom, hydroxy, halogen, substituted or unsubstituted lower alkyl or substituted or unsubstituted amino;
   R⁵¹ represents a hydrogen atom, halogen, substituted or unsubstituted lower alkyl, substituted or unsubstituted lower alkenyl, substituted or unsubstituted lower alkynyl, substituted or unsubstituted lower alkoxy or substituted or unsubstituted amino;
   R⁵² represents substituted or unsubstituted lower alkyl, substituted or unsubstituted aryl or a substituted or unsubstituted aromatic heterocyclic group;
   R⁵³ represents substituted or unsubstituted aryl or a substituted or unsubstituted aromatic heterocyclic group;
   x⁷ and x⁸ are the same or different, each representing a nitrogen atom or CH) (for example,
   5-[6-amino-8-(3-fluorophenyl)-9H-9-purinyl]-1-methyl-1,2-dihydro-2-pyrimidine and the like).
(23) The prophylactic and/or therapeutic agent for diseases accompanied by chronic musculoskeletal pain as described in the above item (1), wherein the compound having an adenosine A_{2A} receptor antagonistic action is a compound represented by formula (XV): 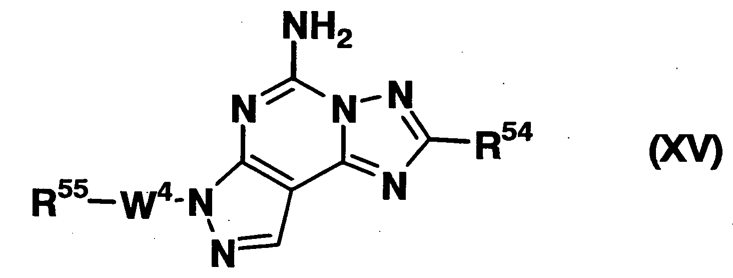 (wherein R⁵⁴ represents a substituted or unsubstituted cycloalkenyl, substituted or unsubstituted aryl or a substituted or unsubstituted aromatic heterocyclic group;
   R⁵⁵ represents substituted or unsubstituted lower alkyl;
   W⁴ represents a single bond or -C(=O)-).
(24) The prophylactic and/or therapeutic agent for diseases accompanied by chronic musculoskeletal pain as described in the above item (1), wherein the compound having an adenosine A_{2A} receptor antagonistic action is a compound represented by formula (XV-A): 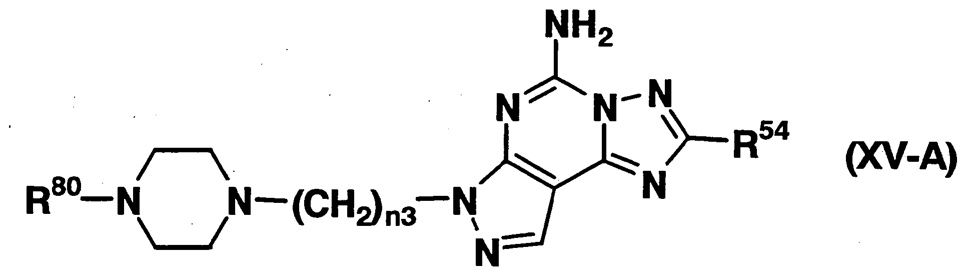 (wherein R⁵⁴ has the same meanings as mentioned above;
   n3 represents an integer of 1 to 4; and
   R⁸⁰ represents substituted or unsubstituted aryl or a substituted or unsubstituted heterocyclic group) (for example, 5-amino-2-(2-furyl)-7-(2-{4-[4-(2-methoxyethoxy)phenyl]-piperazinyl}ethyl)pyrazolo[4,3-e]-1,2,4-triazolo[1,5-c]-pyrimidine and the like).
(25) The prophylactic and/or therapeutic agent for diseases accompanied by chronic musculoskeletal pain as described in the above item (1), wherein the compound having an adenosine A_{2A} receptor antagonistic action is a compound represented by formula (XVI): 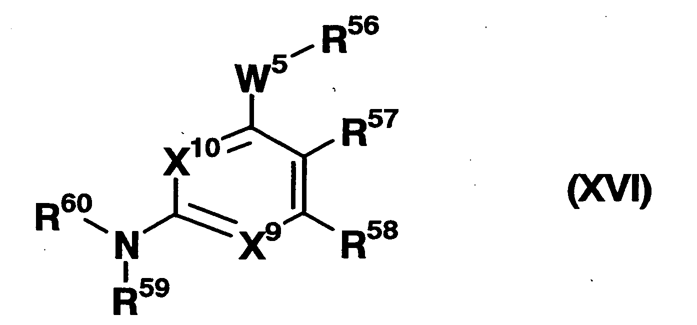 [wherein R⁵⁶ represents a hydrogen atom, halogen, cyano, substituted or unsubstituted lower alkyl, substituted or unsubstituted lower alkenyl, substituted or unsubstituted lower alkynyl or substituted or unsubstituted cycloalkyl;
   R⁵⁷ represents a hydrogen atom, halogen, cyano, nitro, substituted or unsubstituted lower alkyl, substituted or unsubstituted lower alkenyl, substituted or unsubstituted lower alkynyl or substituted or unsubstituted aryl;
   R⁵⁸ represents substituted or unsubstituted lower alkyl, substituted or unsubstituted aryl or a substituted or unsubstituted aromatic heterocyclic group;
   R⁵⁹ and R⁶⁰ are the same or different, each representing a hydrogen atom, substituted or unsubstituted aryl or a substituted or unsubstituted aromatic heterocyclic group;
   W⁵ represents a single bond, -S-, -N(R⁶¹)- (wherein R⁶¹ represents a hydrogen atom or substituted or unsubstituted lower alkyl), -CH₂CH₂-, -CH=CH-, -C≡C- or -O-; and
   X⁹ and X¹⁰ are the same or different; each representing a nitrogen atom or CH].
(26) The prophylactic and/or therapeutic agent for diseases accompanied by chronic musculoskeletal pain as described in the above item (1), wherein the compound having an adenosine A_{2A} receptor antagonistic action is a compound represented by formula (XVII): 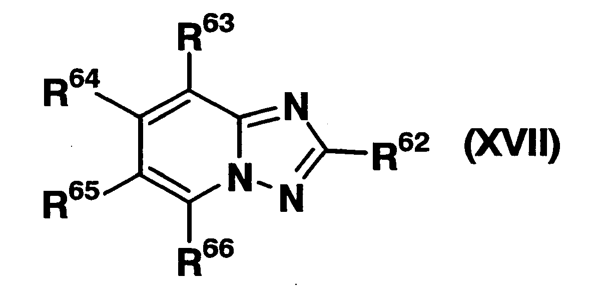 (wherein R⁶² represents substituted or unsubstituted lower alkoxy, substituted or unsubstituted aryl or a substituted or unsubstituted aromatic heterocyclic group:
   R⁶³ and R⁶⁵ are the same or different, each representing a hydrogen atom, cyano or phenylsulfonyl;
   R⁶⁴ represents a hydrogen atom, halogen, substituted or unsubstituted aryl, a substituted or unsubstituted aromatic heterocyclic group or substituted or unsubstituted amino;
   R⁶⁶ represents substituted or unsubstituted amino) (for example, 2-(4,5-dihydrofuran-2-yl)-7-m-tolyl-[1,2,4]triazolo[1,5-a]pyrimidin-5-ylamine and the like).
(27) The prophylactic and/or therapeutic agent for diseases accompanied by chronic musculoskeletal pain as described in the above item (1), wherein the compound having an adenosine A_{2A} receptor antagonistic action is a compound represented by formula (XVIII): 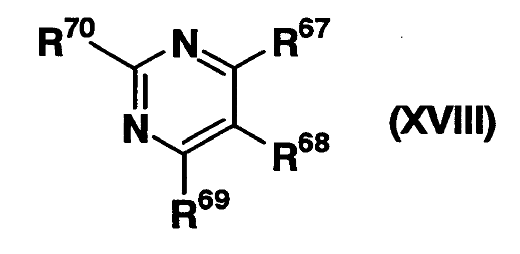 (wherein R⁶⁷ represents substituted or unsubstituted aryl or a substituted or unsubstituted aromatic heterocyclic group;
   R⁶⁸ represents a substituted or unsubstituted aromatic heterocyclic group;
   R⁶⁹ and R⁷⁰ are the same or different, each representing a hydrogen atom or substituted or unsubstituted amino).
(28) The prophylactic and/or therapeutic agent for diseases accompanied by chronic musculoskeletal pain as described in the above item (1), wherein the compound having an adenosine A_{2A} receptor antagonistic action is a compound represented by formula (XIX): 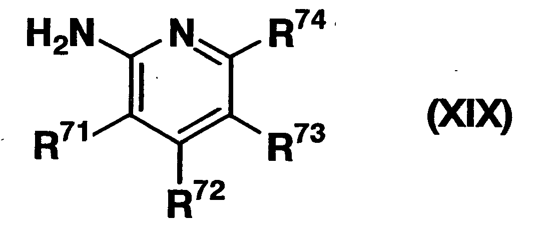 (wherein R⁷¹ represents cyano, carboxy or substituted or unsubstituted carbamoyl;
   R⁷² represents a hydrogen atom, hydroxy, substituted or unsubstituted lower alkoxy, substituted or unsubstituted aryl or a substituted or unsubstituted aromatic heterocyclic group; R⁷³ and R⁷⁴ are the same or different, each representing substituted or unsubstituted aryl or a substituted or unsubstituted heterocyclic group).
(29) The prophylactic and/or therapeutic agent for diseases accompanied by chronic musculoskeletal pain as described in the above item (1), wherein the compound having an adenosine A_{2A} receptor antagonistic action is a compound represented by formula (XX): 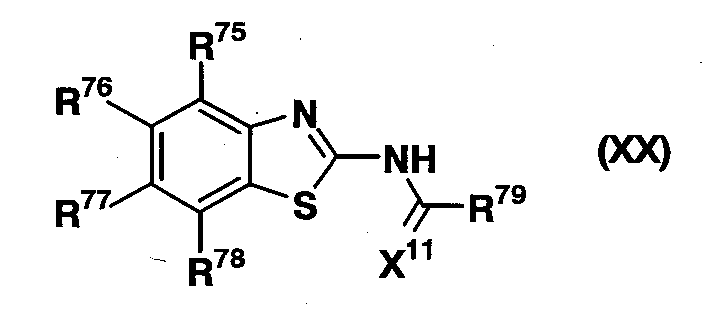 [wherein R⁷⁵ represents a hydrogen atom, hydroxy, halogen, benzyloxy, trifluoromethyloxy, substituted or unsubstituted lower alkyl or substituted or unsubstituted lower alkoxy;
   R⁷⁶ and R⁷⁷ are the same or different, each representing hydroxy, halogen, substituted or unsubstituted lower alkyl or substituted or unsubstituted lower alkoxy;
   R⁷⁸ represents a hydrogen atom, halogen, carboxy, substituted or unsubstituted lower alkyl, substituted or unsubstituted lower alkenyl, substituted or unsubstituted lower alkanoyl, substituted or unsubstituted lower alkoxycarbonyl, substituted or unsubstituted aryl or a substituted or unsubstituted heterocyclic group;
   -C(=X¹¹)- represents -C(=O)-, -C(=S)- or -CH₂-]
(30) The prophylactic and/or therapeutic agent for diseases accompanied by chronic musculoskeletal pain as described in the above item (1), wherein the compound having an adenosine A_{2A} receptor antagonistic action is (-)-(11S,2'R)-α-2-piperidinyl-2,8-bis(trifluoromethyl)-4-quinoline-methanol.
(31) The prophylactic and/or therapeutic agent for diseases accompanied by chronic musculoskeletal pain as described in the above item (1), wherein the compound having an adenosine A_{2A} receptor antagonistic action is a compound represented by formula (XXI) : 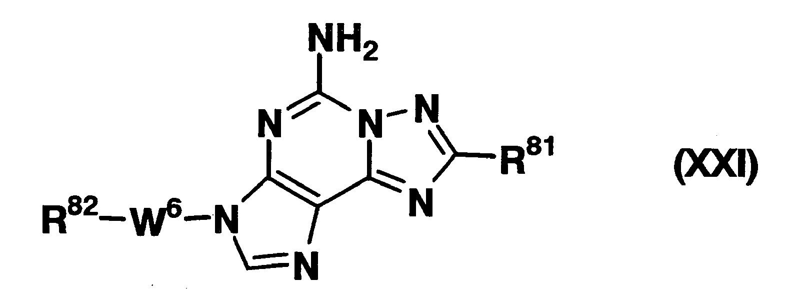 (wherein R⁸¹ represents substituted or unsubstituted aryl, substituted or unsubstituted cycloalkenyl or a substituted or unsubstituted heterocyclic group;
   w⁶ represents a single bond or -C(=O)-; and
   R⁸² represents substituted or unsubstituted lower alkyl).
(32) The prophylactic and/or therapeutic agent for diseases accompanied by chronic musculoskeletal pain as described in the above item (1), wherein the compound having an adenosine A_{2A} receptor antagonistic action is a compound represented by formula (XXI-A): 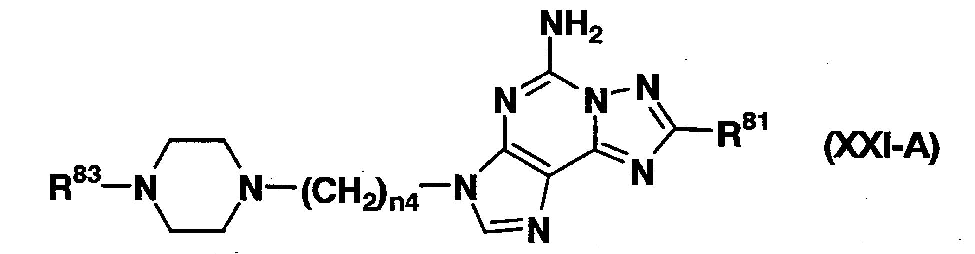 (wherein R⁸¹ has the same meanings as mentioned above;
   n4 represents an integer of 1 to 4;
   R⁸³ represents substituted or unsubstituted aryl or a substituted or unsubstituted heterocyclic group) (for example, 5-amino-2-(2-furyl)-7-(2-{4-[4-(2-methoxyethoxy)phenyl]-piperazinyl}ethyl)imidazo[4,3-e]-1,2,4-triazolo[1,5-c]-pyrimidine and the like).
(33) The prophylactic and/or therapeutic agent for diseases accompanied by chronic musculoskeletal pain as described in any of the above items (1) to (32), wherein the diseases accompanied by chronic musculoskeletal pain is fibromyalgia syndrome.
(34) The prophylactic and/or therapeutic agent for diseases accompanied by chronic musculoskeletal pain as described in any of the above items (1) to (32), wherein the diseases accompanied by chronic musculoskeletal pain is a disease relating to fibromyalgia syndrome.
(35) The prophylactic and/or therapeutic agent for diseases accompanied by chronic musculoskeletal pain as described in the above items (34), wherein the disease relating to fibromyalgia syndrome is one selected from fibrositis, chronic fatigue syndrome, myofascial pain syndrome, diffuse myofascial pain syndrome, generalized fibrisitis, soft tissue rheumatism, non-articular rheumatism, chronic rheumatoid arthritis, primary fibromyalgia syndrome, concomitant fibromyalgia syndrome, idiopathic muscle pain syndrome, chronic widespread musculoskeletal pain, lower back pain, Lyme disease accompanied by fibromyalgia syndrome, generalized tendomyopathy, and temporomansibular joint disorder.
(36) A method of preventing and/or treating diseases accompanied by chronic musculoskeletal pain which comprises administering an effective amount of a compound having an adenosine A_{2A} receptor antagonistic action or a pharmaceutically acceptable salt thereof.
(37) Use of a compound having an adenosine A_{2A} receptor antagonistic action or a pharmaceutically acceptable salt thereof for the manufacture of a prophylactic and/or therapeutic agent for diseases accompanied by chronic musculoskeletal pain.

### Effect of the Invention

According to the present invention, a prophylactic and/or therapeutic agent for diseases accompanied by chronic musculoskeletal pain which comprises as an active ingredient a compound having an adenosine A_{2A} receptor antagonistic action or a pharmaceutically acceptable salt thereof, is provided.

### Brief Description of Drawings

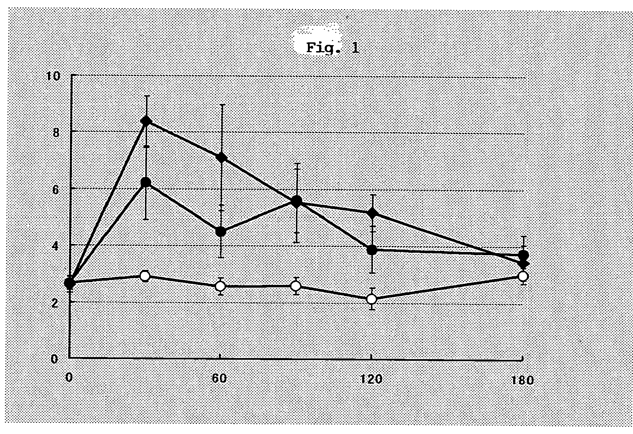

Fig. 1 shows the effect of Compound 2 for improvement of hyperalgesia in a model of hyperalgesia induced by an acidic physiological saline. The y axis indicates the pain threshold (g) and the x axis indicates the elapsed time (minutes) after administration of Compound 2 or a solvent.

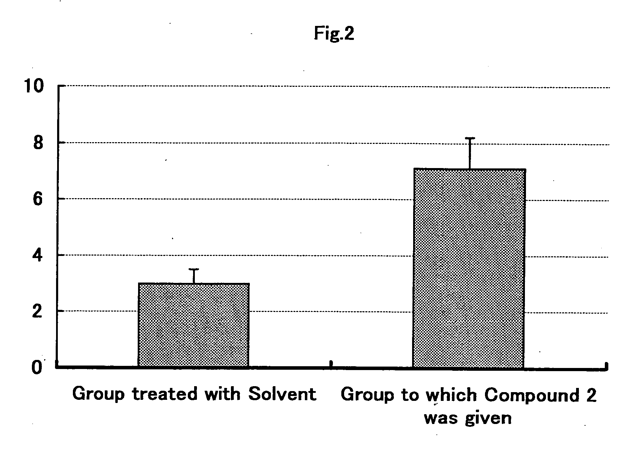

Fig. 2 shows the effect of Compound 2 for preventing the development of hyperalgesia in a model of hyperalgesia induced by an acidic physiological saline. The y axis indicates the pain threshold (g); the left bar shows the results in the group treated with Solvent; and the right bar shows the results in the group to which Compound 2 was administered.

### Explanation of Reference Signs in Drawings

Fig. 1:
   -◆-: Group to which 10mg/kg of Compound 2 was given
   -•-: Group to which 5mg/kg of Compound 2 was given
   -o-: Group treated with Solvent

### Best Mode for Carrying Out the Invention

The diseases accompanied by chronic musculoskeletal pain which are prevented or treated in the present invention include, for example, fibromyalgia syndrome (FMS) and diseases related thereto include, for examples, fibrositis, chronic fatigue syndrome (CFS), myofascial pain syndrome (MFPS), diffuse myofascial pain syndrome, generalized fibrisitis, soft tissue rheumatism, non-articular rheumatism, chronic rheumatoid arthritis, primary fibromyalgia syndrome (PFS), concomitant fibromyalgia syndrome, idiopathic muscle pain syndrome, chronic widespread musculoskeletal pain, low back pain, Lyme disease accompanied by fibromyalgia syndrome, generalized tendomyopathy, temporomansibular joint disorder (TMJD), and the like.

The adenosine A_{2A} receptor antagonistic action means the inhibition, suppression or stopping of at least one of physiological effects involving adenosine, for example, by binding to an adenosine A_{2A} receptor or by obstructing or preventively inhibiting the binding of adenosine to an adenosine A_{2A} receptor.

Compounds having an adenosine A_{2A} receptor antagonistic action are not particularly limited as far as they have an adenosine A_{2A} receptor antagonistic action, and include those as described in US 5,484,920, US 5,703,085, WO 92/06976, WO 94/01114, US 5,565,460, WO 98/42711, WO 00/17201, WO 99/43678, WO 99/26627, WO 01/92264, WO 99/35147, WO 00/13682, WO 00/13681, WO 00/69464, WO 01/40230, WO 01/02409, WO 01/02400, EP 1054012, WO 01/62233, WO 01/17999, WO 01/80893, WO 02/14282, WO 01/97786, WO 03/032996, WO 03/048163, WO 03/049164, WO 03/049165, and so on. Specifically, the following compounds are exemplified: the above-mentioned compounds represented by formulae (I), (V) to (XXI), (XV-A) and (XXI-A)(hereinafter, referred to as Compounds (I), Compounds (V) to (XXI), Compounds (XV-A) and Compounds (XXI-A), respectively), and (-)-(11S,2'R)-α-2-piperidinyl-2,8-bis(trifluoromethyl)-4-quinolinemethanol. In particular, xanthine derivatives such as Compounds (I) or (X), [1,2,4]triazolo[1,5-c]pyrimidine derivatives such as Compounds (V), (VI) or-(Ix), and [1,2,4]triazolo[1,5-a]pyrimidine derivatives such as Compounds (VII) or (VIII), are preferred. Particularly, the following compounds are preferred.

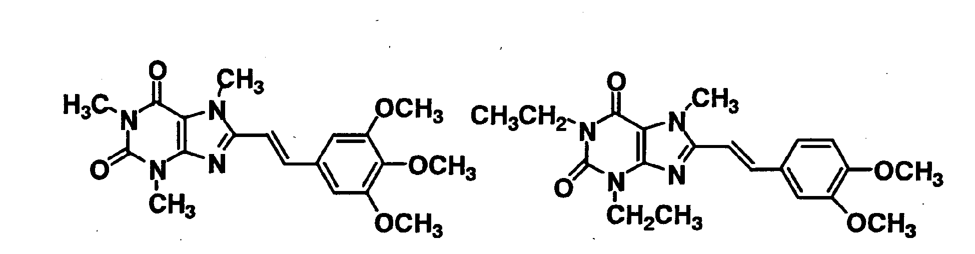

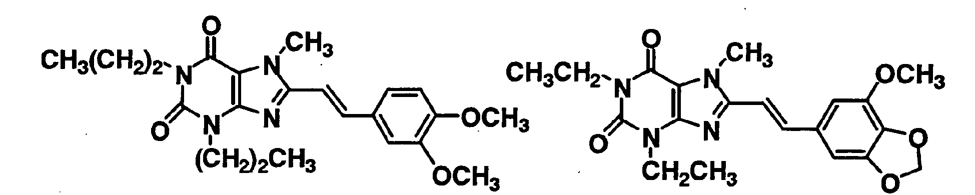

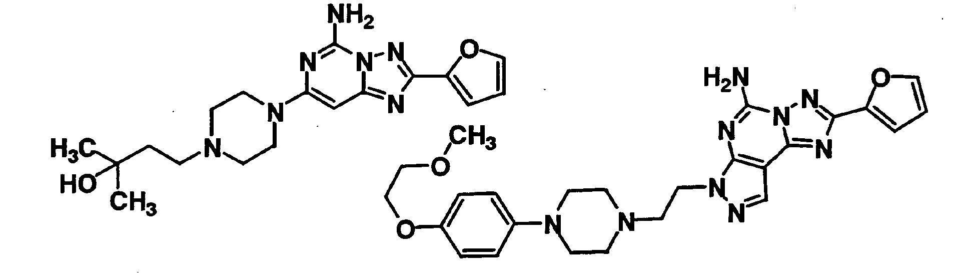

In the definition of the respective groups in formulae (I) to (XXI), formula (XV-A) and formula (XXI-A), the lower alkyl moiety of lower alkyl, hydroxy(lower)alkyl, lower alkoxy, lower alkoxycarbonyl and lower alkanoyl means, for example, straight or branched chain alkyl of 1 to 8 carbon atoms, specifically including methyl, ethyl, propyl, isopropyl, butyl, isobutyl, sec-butyl, tert-butyl, pentyl, neopentyl, hexyl, heptyl, octyl, and the like.

The lower alkenyl means, for example, straight or branched chain alkenyl of 2 to 8 carbon atoms, specifically including vinyl, allyl, methacryl, crotyl, 3-butenyl, 2-pentenyl, 4-pentenyl, 2-hexenyl, 5-hexenyl, 2-heptenyl, 2-octenyl, and the like.

The lower alkynyl means, for example, straight or branched chain alkyl of 2 to 8 carbon atoms, specifically including ethynyl, propargyl, 2-butynyl, 3-butynyl, 2-pentynyl, 4-pentynyl, 2-hexynyl, 5-hexynyl, 4-methyl-2-pentynyl, 2-heptynyl, 2-octyny, and the like.

The cycloalkyl means, for example, those of 3 to 8 carbon atoms, specifically including cyclopropyl, cyclobutyl, cyclopentyl, cyclohexyl, cycloheptyl, cyclooctuyl, and the like.

The cycloalkenyl means, for example, those of 4 to 8 carbon atoms, specifically including cyclobutenyl, cyclopentenyl, cyclohexenyl, cycloheptenyl, cyclooctenyl, and the like.

The halogen means fluorine, chlorine, bromine and iodine atoms.

The aryl means, for example, those of 6 to 14 carbon atoms, specifically including phenyl, naphthyl, anthryl, and the like.

The aromatic heterocyclic group means, for example, 5-or 6-membered monocyclic aromatic heterocyclic group containing at lease one atom selected from nitrogen atom, oxygen atom and sulfur atom, bicyclic or tricyclic condensed aromatic heterocyclic groups condensed with a 3- to 8-membered ring and containing at lease one atom selected from nitrogen atom, oxygen atom and sulfur atom, specifically including pyridyl, pyrazinyl, pyrimidinyl, pyridazinyl, oxopyridazinyl, quinolyl, isoquinolyl, phthalazinyl, quinazolinyl, quinoxalinyl, naphthylidinyl, cinnolinyl, pyrrolyl, pyrazolyl, imidazolyl, triazinyl, triazolyl, tetrazolyl, thienyl, furyl, thiazolyl, isothiazolyl, oxazolyl, isoxazolyl, oxadizaolyl, thiadiazolyl, oxooxadiazolyl, indolyl, isoindolyl, indazolyl, 2-oxobenzimidazolyl, benzofuryl, benzothienyl, benzimidazolyl, benzotriazolyl, benzothiazolyl, benzoxazolyl, purinyl, dibennzofuranyl, imidazo[1,2-a]pyridyl, and the like.

The heterocyclic group includes, for example, in addition to the above-defined aromatic heterocyclic groups, alicyclic heterocyclic groups. The alicyclic heterocyclic group means, for example, 5- or 6-memberedmonocyclic alicyclic heterocyclic groups containing at least one atom selected from nitrogen atom, oxygen atom and sulfur atom, bicyclic or tricyclic condensed alicyclic heterocyclic groups condensed with a 3- to 8-membered ring and containing at lease one atom selected from nitrogen atom, oxygen atom and sulfur atom, specifically including pyranyl, thiopyranyl, pyrrolidinyl, piperidino, piperazinyl, piperidinyl, imidazolidinyl, thiazolidinyl, morpholino, morpholinyl, thiomorpholino, thiomorpholinyl, homopiperidino, homopiperazinyl, tetrahydropyridinyl, dihydroisoquinolyl, tetrahydroquinolyl, tetrahydroisoquinolyl, oxazolinyl, oxazolidinyl, oxooxazolidinyl, oxadiazolinyl, oxolanyl, tetrahydrofuranyl, tetrahydropyranyl, dihydrobenzofuranyl, oxopiperazinyl, 2-oxopyrrolidinyl, dioxolanyl, 1,3-benzodioxolyl, 1,4-benzodioxanyl, 3,4-dihydro-2H-1,5-benzodioxepinyl, benzopyranyl, benzodihydropyranyl, perhydrodiazepinyl, perhydrodiazocinyl, perhydrodiazoninyl, and the like.

The heterocycle formed by "A" together with the adjacent 2 carbon atoms means, for example, 5- or 6-membered monocyclic heterocycles containing at least one atom selected from nitrogen atom, oxygen atom and sulfur atom, bicyclic or tricyclic condensed heterocycles condensed with a 3- to 8-membered ring and containing, for example at lease one atom selected from nitrogen atom, oxygen atom and sulfur atom, specifically including pyrrole, pyran, thiopyran, pyridine, thiazole, imidazole, pyrimidine, triazine, indole, quinoline, benzothiazole, pyrroline, tetrahydropyridine, tetrahydropyrazine, tetrahydroquinoline, tetrahydroisoquinoline, and the like.

The carbocycle formed by "A" together with the adjacent 2 carbon atoms means, for example, cycloalkenes of 4 to 8 carbon atoms, specifically including cyclobutene, cyclopentene, cyclohexene, cycloheptene, cyclooctene, and the like.

The carbocycle formed together with the adjacent carbon atoms means, for example, cycloalkanes and cyclalkenes of 4 to 8 carbon atoms, specifically including cyclobutane, cyclopentane, cyclohexane, cycloheptane, cyclooctane, cyclobutene, cyclopentene, cyclohexene, cycloheptene, cyclooctene, and the like.

The substituents (A) in the substituted lower alkyl, substituted lower alkoxy, substituted lower alkanoyl, substituted lower alkoxycarbonyl, substituted lower alkenyl and substituted lower alkynyl, are the same or different in number of 1 to 3, specifically including hydroxy, cyano, nitro, carboxy, carbamoyl, amino, benzyloxy, phenoxy, halogen, substituted or unsubstituted lower alkoxy, cycloalkyl, lower alkanoyl, lower alkoxycarbonyl, lower alkylamino, di(lower) alkylamino, substituted or unsubstituted aryl, substituted or unsubstituted heterocyclic group, and the like.

The halogen, cycloalkyl, aryl and heterocyclic groups exemplified in definition of the substituent (A) each has the same meanings as mentioned above; the lower alkyl moiety of the lower alkoxy, lower alkanoyl, lower alkoxycarbonyl, lower alkylamino and di(lower) alkylamino has the same meanings as the above-mentioned lower alkyl, wherein the two lower alkyl portions of di (lower) alkylamino may be the same or different.

The substituents in the substituted aryl and substituted heterocyclic groups exemplified in definition of the substituent (A) may be the same or different in number of 1 to 3, and specifically include those exemplified in the substituent (C) as mentioned below.

The substituents (a) in the substituted lower alkoxy exemplified in the substituent (A) may be the same or different in number of 1 to 3, and specifically include halogen, hydroxy, amino, carbonyl, azido, lower alkoxy, lower alkoxycarbonyl, and the like. The halogen exemplified in the substituent (a) has the same meanings as mentioned above, and the lower alkyl moiety of the lower alkoxy and lower alkoxycarbonyl has the same meanings as lower alkyl mentioned above.

The substituents (B) in the substituted amino and substituted carbamoyl may be the same or different in number of 1 to 2, and specifically include substituted or unsubstituted lower alkyl, substituted or unsubstituted lower alkoxy, and the like.

The lower alkyl and lower alkoxy exemplified in the substituent (B) each has the same meanings as mentioned above, and the substituents in the substituted lower alkyl and substituted lower alkoxy exemplified in the substituent (B) are the same or different in number of 1 to 3, and specifically include those exemplified in the substituent (a) as mentioned above.

The substituents (C) in the substituted cycloalkyl, substituted cycloalkenyl, substituted carbocycle formed together with the adjacent carbon atom, substituted carbocycle formed by "A" together with the adjacent two carbon atoms, substituted aryl, substituted phenyl, substituted naphthyl, substituted heterocyclic group, substituted aromatic heterocyclic group and substituted heterocycle formed by "A" together with the adjacent two carbon atoms, may be the same or different in number of 1 to 3, and include, for example, hydroxy, nitro, amino, carboxy, sulfo, trifluoromethyl, halogen, lower alkyl, lower alkenyl, lower alkynyl, substituted or unsubstituted lower alkoxy, lower alkylamino, di(lower) alkylamino, substituted or unsubstituted aryl, aryloxy, aralkyl, aralkyloxy, lower alkanoyl, lower alkanoyloxy, aroyl, aroyloxy, arylalkanoyloxy, lower alkoxycarbonyl, lower alkylcarbamoyl, di(lower) alkylcarbamoyl, lower alkoxysulfonyl, lower alkylsulfamoyl, di(lower) alkylsulfamoyl, and the like.

The lower alkyl moiety in the lower alkyl, lower alkoxy, lower alkylamino, di (lower) alkylamino, lower alkanoyl, lower alkanoyloxy, lower alkoxycarbonyl, lower alkylcarbamoyl, di(lower) alkylcarbamoyl, lower alkoxysulfonyl, lower alkylsulfamoyl, and di(lower) alkylsulfamoyl exemplified in the substituents (C) has the same meanings as the above-mentioned lower alkyl, and the halogen, lower alkenyl and lower alkynyl each has the same meanings as mentioned above. The two lower alkyl portions of the di(lower) alkylamino, di(lower) alkylcarbamoyl and di(lower) alkylsulfamoyl may be the same or different. The aryl moiety of the aryl and aryloxy is the same as that of the above-mentioned aryl. The aralkyl moiety of the aralkyl and aralkyloxy is, for example, benzyl, phenethyl, and the like. The aroyl moiety of the aroyl and aroyloxy is, for example, benzoyl, naphthoyl, and the like. The arylalkyl moiety of the arylalkanoyloxy is, for example, benzyl, phenethyl, and the like.

The substituents (c) in the substituted lower alkoxy and substituted aryl exemplified in the substituent (C) may be the same or different in number of 1 to 3, and include, for example, those exemplified in the above-mentioned substituents (a).

The pharmaceutically acceptable salt of the compound having an adenosine A_{2A} receptor antagonistic action includes, for example, pharmaceutically acceptable acid addition salts, metal salts, ammonium salts, organic amine addition salts, amino acid addition salts, and the like.

The pharmaceutically acceptable acid addition salt of the compound having an adenosine A_{2A} receptor antagonistic action includes, for example, inorganic acid salts such as hydrochloride, sulfate, phosphate; organic acid salts such as acetate, maleate, fumarate, tartrate, citrate, methansulfonate, and the like. The pharmaceutically acceptable metal salt includes, for example, alkali metal salts such as sodium salt, potassium salt; alkaline earth metal salts such as magnesium salt, calcium salt; aluminum salt, zinc salt, and the like. The pharmaceutically acceptable ammonium salt includes, for example, salts with ammonium, tetramethylammonium, and the like. The pharmaceutically acceptable organic amine addition salt include addition salts with morpholine, piperidine, and the like. The pharmaceutically acceptable amino acid addition salt includes addition salts with lysine, glycine, phenylalanine, and the like.

Compounds (I), Compounds (V) to (XXI), Compounds (XV-A) and Compounds (XXI-A) may be produced according to the processes as disclosed in the following documents or analogous processes: JP-B 47-26516; J. Med. Chem., 34, p.1431 (1991) ; J. Med. Chem., 36, p.1333 (1993) ; WO 92/06976; JP-A 6-211856; JP-A 6-239862; WO 95/23165; JP-A 6-16559; WO 94/01114; WO 99/12546; WO 99/35147; US 5,484,920; US 5,703,085; WO 92/06976; WO 94/01114; US 5,565,460; WO 98/42711; WO 00/17201; WO 99/43678; WO 99/26627; WO 01/92264; WO 99/35147; WO 00/13682; WO 00/13681; WO 00/69464; WO 01/40230; WO 01/02409; WO 01/02400; EP 1054012; WO 01/62233; WO 01/17999; WO 01/80893; WO 02/14282; WO 01/97786; WO 03/032996; WO 03/048163; WO 03/049164; and WO 03/049165 and the like. The objective compounds in the respective processes may be isolated and purified according to conventional ways usually employed in organic syntheses, for example, filtration, extraction, washing, drying, concentration, recrystallization, a variety of chromatography.

In order to obtain the salts of compounds having an adenosine A_{2A} receptor antagonistic action such as Compounds (I), Compounds (V) to (XXI), Compounds (XV-A) and Compounds (XXI-A), when the compounds having an adenosine A_{2A} receptor antagonistic action such as Compounds (I), Compounds (V) to (XXI), Compounds (XV-A) and Compounds (XXI-A), respectively can be obtained in the forms of salts, they may be purified as such, and when obtained in the free forms, the compounds having an adenosine A_{2A} receptor antagonistic action such as Compounds (I), Compounds (V) to (XXI), Compounds (XV-A) and Compounds (XXI-A), respectively may be dissolved or suspended in a suitable solvent, to which an acid or base is added to form the salts.

In addition, the compounds having an adenosine A_{2A} receptor antagonistic action, such as Compounds (I), Compounds (V) to (XXI), Compounds (XV-A) and Compounds (XXI-A), or pharmaceutically acceptable salts thereof may sometimes exist in a form of adduct with water or a variety of solvents; these adducts can be used in the present invention as prophylactic and/or therapeutic agents for diseases accompanied by chronic musculoskeletal pain.

Some of the compounds having an adenosine A_{2A} receptor antagonistic action, such as Compounds (I), Compounds (V) to (XXI), Compounds (XV-A) and Compounds (XXI-A) may exist as stereoisomers such as optical isomers; all of possible stereoisomers and their mixtures can be used in the present invention as prophylactic and/or therapeutic agents for diseases accompanied by chronic musculoskeletal pain.

Specific examples of Compounds (I) are shown in Table 1.

**Table 1**

| Compound | No. |
|---|---|
| 1 | 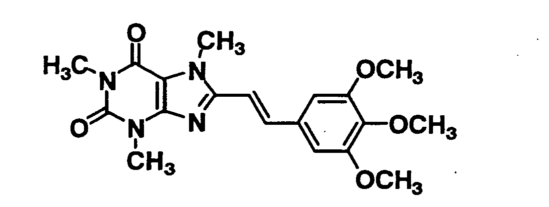 |
| 2 | 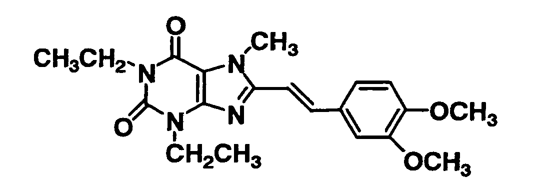 |
| 3 | 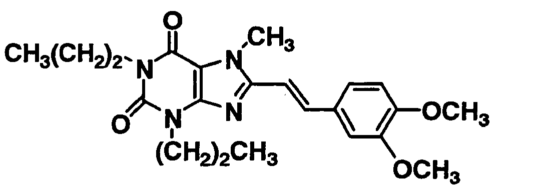 |
| 4 | 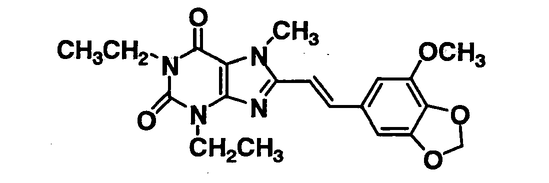 |

Effect of the present invention will be explained specifically by the following Test Examples.

### Test Example 1: Effect for pain relief in a hyperalgesia model of rat

An acidic physiological saline-induced hyperalgesia model, which is known as a model of diseases accompanied by chronic musculoskeletal pain was made according to the method described in Muscle & Nerve 24, p.37-46 (2001) with a slight modification.

In this test, male SD rats (Sprague-Dawley rats; 6 weeks of age at the start of the test) were employed. Under ether anesthesia, 100µl of physiological saline adjusted to pH 4 with acetic acid was injected to the rat's right gastrocnemius muscle twice at an interval of 5 days. At 10th and 11th days from the 2nd injection, the following test was conducted. The pain threshold (g) was calculated by the following method, and rats which showed 50% pain threshold of less than 4 g (hyperalgesia rats induced with an acidic physiological saline) were employed in the test, using 6 rats for each treatment-group. In this connection, the pain threshold of normal rats was approximately 11 g.

The above hyperalgesia rats induced with an acidic physiological saline were placed in a stainless steel cage (750width x 210depth x 170height in mm) and accommodated thereto for at least 20 minutes. Thereafter, the pain threshold (g) was determined prior to administration of the test compound (0 hour) and 0.5 hour, 1 hour, 1.5 hours, 2 hours and 3 hours after the administration.

The test compound was used as a suspension in 0.5% methylcellulose (0.5% MC) aqueous solution and orally administered to the hyperalgesia rats induced with an acidic physiological saline at a volume of 5mL/kg (groups treated with Test Compound). A separate group of rats showing hyperalgesia induced with an acidic physiological saline each received orally 0.5% MC aqueous solution alone at a volume of 5mL/kg, which was used as a group treated with Solvent.

Hyperalgesia was evaluated by the von Frey test, and the results were indicated by the thresholds (g). That is, using a von Frey filament (trade name: touch test sensory evaluator; model no. 58011), the hyperalgesia rat induced with an acidic physiological saline was given a mechanical stimulus at the right leg to which an acidic physiological saline had been injected, and the load to withdraw the leg was measured. The pain threshold (g) was calculated according to the W.J. Dixon' s up-down method (Annual Review of Pharmacology and Toxicology, 20, p.441-462 (1980)). The results are shown in Fig. 1.

From the above results, the followings were elucidated.

In the group treated with Test Compound to which Compound 2 was orally administered (5 and 10 mg/kg), the pain threshold observed in the hyperalgesic rats induced with an acidic physiological saline was greatly increased in comparison with that of the group treated with Solvent. This indicates that the hyperalgesia was markedly improved by administration of Compound 2.

### Test Example 2: Preventive effect on development of hyperalgesia in a rat model

The following experiment was carried out according to the method as described in Test Example 1.

In this test, male SD rats (Sprague-Dawley rats; 6 weeks of age at the start of the test) were employed as 10 rats for each treatment-group. Under ether anesthesia, 100µl of physiological saline adjusted to pH 4 with acetic acid was injected to the rat's right gastrocnemius muscle twice at an interval of 5 days. Thirty minutes after the 2nd injection and once daily thereafter, a test compound or solvent was administered repeatedly at a volume of 5 ml/kg to the above rats for a total of 10 days.

The test compound was used as a suspension in 0.5% MC aqueous solution and orally administered (a group treated with Test Compound), and in a group to which a solvent was administered 0.5% MC aqueous solution alone was orally administered (a group treated with Solvent).

On the day following the final administration, the pain threshold (g) was measured in the same manner as the von Frey test as described in Test Example 1. The above rats were placed in a stainless steel cage (750width x 210depth x 170height in mm) and accommodated thereto for at least 20 minutes; after that the measurement was conducted. The results are shown in Fig. 2. From the above results, the followings were elucidated.

The pain threshold similarly determined in normal rats was approximately 11 g. In the group treated with Solvent in which group a solvent only was administered repeatedly, the threshold was greatly decreased to the value indicating the development of hyperalgesia. On the other hand, in the group treated with Test Compound in which group Compound 2 (3 mg/kg) was administered repeatedly, no decrease of the pain threshold was observed, indicating that the development of hyperalgesia was prevented.

From the above test results, it was found that a compound having an adenosine A_{2A} receptor antagonistic action or a pharmaceutically acceptable salt thereof has effects of improving fully developed hyperalgesia and preventing the development of hyperalgesia. In other words, it was suggested that the compound having an adenosine A_{2A} receptor antagonistic action or a pharmaceutically acceptable salt thereof is useful as a prophylactic and/or therapeutic agent for diseases exhibiting chronic musculoskeletal pain.

The compound having an adenosine A_{2A} receptor antagonistic action or a pharmaceutically acceptable salt thereof may be used as such or in a variety of pharmaceutical formulations. The pharmaceutical formulations can be produced by homogeneously mixing a compound having an adenosine A_{2A} receptor antagonistic action or a pharmaceutically acceptable salt thereof with a pharmaceutically acceptable carrier. These pharmaceutical formulations are desirably in a dosage unit form suitable for rectal, oral or parenteral (including subcutaneous, intravenous and intramuscular) administration.

In preparing a composition in an orally administrable formulation, certain useful pharmaceutically acceptable carriers may be used. For example, oral liquid preparations such as suspensions and syrups may be prepared using water, saccharides such as sucrose, sorbitol or fructose, glycols such as polyethylene glycol or propylene glycol, oils such as sesame oil, olive oil or soybean oil, preservatives such as p-hydroxybenzoic acid ester, flavors such as strawberry flavor or peppermint, and the like. Powders, pills, capsules and tablets may be prepared with excipients such as lactose, glucose, sucrose or mannitol, disintegrators such as starch or sodium alginate, lubricants such as magnesium stearate or talc, binders such as polyvinyl alcohol, hydroxypropyl cellulose or gelatin, surfactants such as fatty acid ester, plasticizers such as glycerin, and the like. Tablets and capsules are most useful unit oral dosage preparations since they can easily be administered. In preparing tablets or capsules, solid pharmaceutical carriers are employed.

In addition, preparations for injection may be prepared with a carrier comprising distilled water, salt solutions, glucose solutions or a mixture of saline and glucose solution. In this operation, the preparation is prepared into a solution, suspension or dispersion using an auxiliary agent according to a conventional manner.

The compounds having an adenosine A_{2A} receptor antagonistic action or pharmaceutically acceptable salts thereof can be administered orally in the formulations as mentioned above or parenterally as injections. Though the effective dose and the frequency of administration vary with a mode of administration, the age, body weight, conditions of patient, and so on, they may be administered at a single dose or divided doses of 1-100mg/60kg/day, preferably 1-20mg/60kg/day.

The embodiment of the present invention will be explained according to the following examples.

### Example 1 Tablets

Tablets comprising the following ingredients are prepared.

To a mixture of 40 g of Compound 1, 286.8 g of lactose and 60 g of potato starch is added 120 g of 10% aqueous solution of hydroxypropyl cellulose. The mixture is then kneaded in a conventional way, granulated, dried, and sized to give granules for tableting. Magnesium stearate (1.2 g) is added thereto, and the mixture is applied to tableting in a tableting machine (Kikusui Type RT-15) with a punch of 8 mm in diameter to give tablets (containing 20 mg of active ingredient per tablet).

| Prescription Compound 1 | | 20 mg |
|---|---|---|
| | Lactose | 143.4 mg |
| | Potato starch | 30 mg |
| | Hydroxypropyl cellulos | 6 mg |
| | Magnesium stearate | 0.6 mg |
| | | 200 mg |

### Example 2 Capsules

Capsules comprising the following ingredients are prepared in a conventional way.

In a conventional way, 200 g of Compound 2, 995 g of Avicel and 5 g of magnesium stearate were mixed in a conventional way. The mixture is filled in no.4 hard capsules (120 mg of content per capsule) by a capsule filling machine (Zanasi Co.; Type LZ-64) to give capsules (containing 20 mg of active ingredient per capsule)

| Prescription Compound 2 | | 20 mg |
|---|---|---|
| | Avicel | 99.5 mg |
| | Magnesium stearate | 0.5 mg |
| | | 120 mg |

### Example 3 Injections

Injections comprising the following ingredients are prepared in a conventional way.

In 100 g of purified soybean oil is dissolved 1 g of Compound 3, to which is added 12 g of purified egg yolk lecithin and 25 g of glycerin for injection. The mixture is made 1000 mL with distilled water for injection and then kneaded and emulsified in a conventional way. The resulting dispersed solution is sterilely filtered through a disposable membrane filter of 0.2µm, and 2mL each is filled in a glass vial sterilely to give injection preparations (containing 2 mg of active ingredient per vial).

| Prescription Compound 3 | | 2 mg |
|---|---|---|
| | Purified soybean oil | 200 mg |
| | purified egg yolk lecithin | 24 mg |
| | Glycerin for injection | 50 mg |
| | Distilled water for | |
| | injection | 1.72 mL |
| | | 2.00 mL |

### Industrial Applicability

According to the present invention, a prophylactic and/or therapeutic agent for diseases accompanied by chronic musculoskeletal pain which comprises as an active ingredient a compound having an adenosine A_{2A} receptor antagonistic action or a pharmaceutically acceptable salt thereof, is provided.

## Claims

1. A prophylactic and/or therapeutic agent for diseases accompanied by chronic musculoskeletal pain which comprises as an active ingredient a compound having an adenosine A_{2A} receptor antagonistic action or a pharmaceutically acceptable salt thereof.

2. The prophylactic and/or therapeutic agent for diseases accompanied by chronic musculoskeletal pain as claimed in Claim 1, wherein the compound having an adenosine A_{2A} receptor antagonistic action is a xanthine derivative.

3. The prophylactic and/or therapeutic agent for diseases accompanied by chronic musculoskeletal pain as claimed in Claim 1, wherein the compound having an adenosine A_{2A} receptor antagonistic action is a xanthine derivative represented by formula (I): 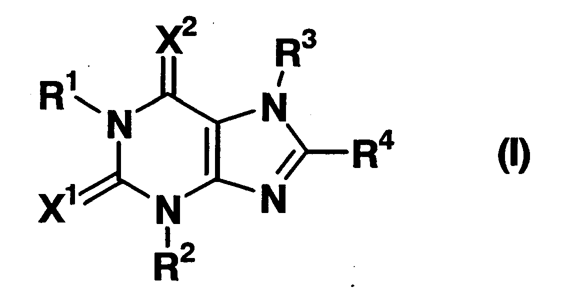 [wherein R¹, R² and R³ are the same or different, each representing a hydrogen atom, lower alkyl, lower alkenyl or lower alkynyl;
R⁴ represents cycloalkyl, -(CH₂)ₙ-R⁵ (in which R⁵ represents substituted or unsubstituted aryl or a substituted or unsubstituted heterocyclic group; and n is an integer of 0 to 4) or formula (II): 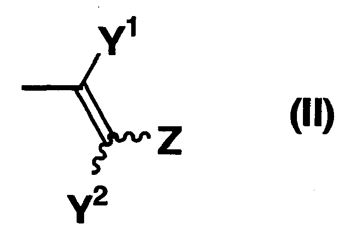 (wherein Y¹ and Y² are the same or different, each representing a hydrogen atom, halogen or lower alkyl; and Z represents substituted or unsubstituted aryl or a substituted or unsubstituted heterocyclic group); and X¹ and X² are the same or different, each representing an oxygen atom or a sulfur atom].

4. The prophylactic and/or therapeutic agent for diseases accompanied by chronic musculoskeletal pain as claimed in Claim 3, wherein X¹ and X² each is an oxygen atom.

5. The prophylactic and/or therapeutic agent for diseases accompanied by chronic musculoskeletal pain as claimed in Claim 3 or 4, wherein R⁴ is a formula (II): 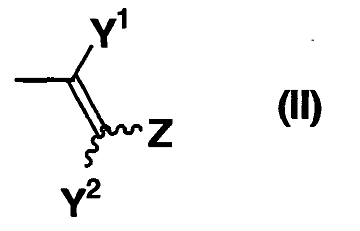 (wherein Y¹, Y² and Z each has the same meanings as mentioned above).

6. The prophylactic and/or therapeutic agent for diseases accompanied by chronic musculoskeletal pain as claimed in Claim 5, wherein Y¹ and Y² each is a hydrogen atom.

7. The prophylactic and/or therapeutic agent for diseases accompanied by chronic musculoskeletal pain as claimed in Claim 5 or 6, wherein Z is substituted or unsubstituted aryl or a group represented by formula (III): 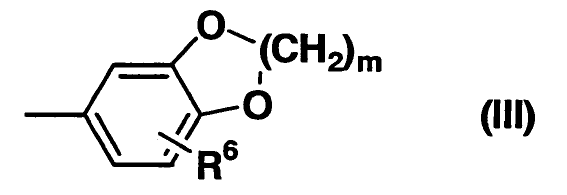 (wherein R⁶ represents a hydrogen atom, hydroxy, lower alkyl, lower alkoxy, halogen, nitro or amino; and m represents an integer of 1 to 3).

8. The prophylactic and/or therapeutic agent for diseases accompanied by chronic musculoskeletal pain as claimed in Claim 5 or 6, wherein Z is a group represented by formula (IV): 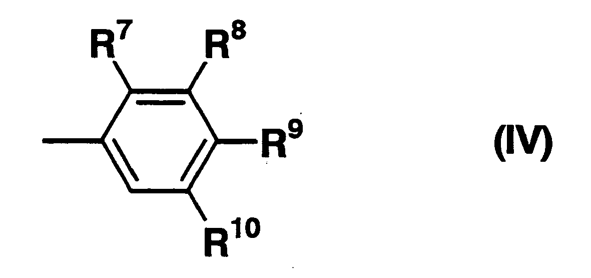 (wherein at least one of R⁷, R⁸ and R⁹ represents lower alkyl or lower alkoxy and the remaining groups represent a hydrogen atom; R¹⁰ represents a hydrogen atom or lower alkyl) or formula (III): 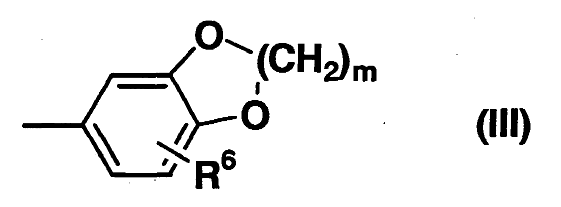 (wherein R⁶ and m each has the same meanings as mentioned above).

9. The prophylactic and/or therapeutic agent for diseases accompanied by chronic musculoskeletal pain as claimed in any of Claims 3 to 8, wherein R¹ and R² each is ethyl.

10. The prophylactic and/or therapeutic agent for diseases accompanied by chronic musculoskeletal pain as claimed in any of Claims 3 to 9, wherein R³ is methyl.

11. The prophylactic and/or therapeutic agent for diseases accompanied by chronic musculoskeletal pain as claimed in Claim 1, wherein the compound having an adenosine. A_{2A} receptor antagonistic action is a compound represented by formula (1):

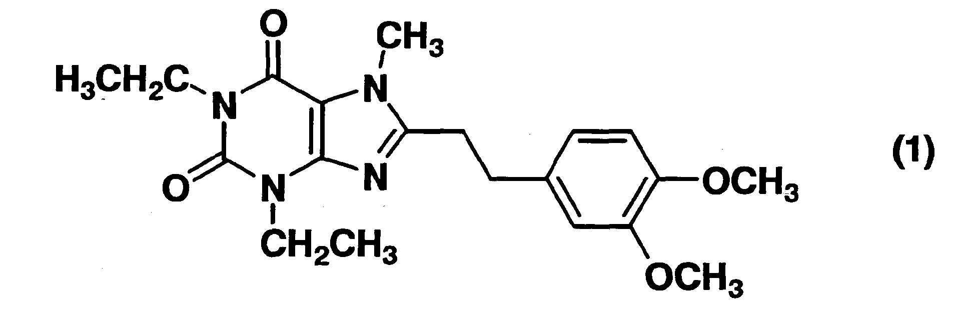

12. The prophylactic and/or therapeutic agent for diseases accompanied by chronic musculoskeletal pain as claimed in any of Claims 1 to 11, wherein the disease accompanied by chronic musculoskeletal pain is fibromyalgia syndrome.

13. The prophylactic and/or therapeutic agent for diseases accompanied by chronic musculoskeletal pain as claimed in any of Claims 1 to 12, wherein the disease accompanied by chronic musculoskeletal pain is a disease relating to fibromyalgia syndrome.

14. The prophylactic and/or therapeutic agent for diseases accompanied by chronic musculoskeletal pain as claimed in Claim 13, wherein the disease relating to fibromyalgia syndrome is one selected from fibrositis, chronic fatigue syndrome, myofascial pain syndrome, diffuse myofascial pain syndrome, generalized fibrisitis, soft tissue rheumatism, non-articular rheumatism, chronic rheumatoid arthritis, primary fibromyalgia syndrome, concomitant fibromyalgia syndrome, idiopathic muscle pain syndrome, chronic widespread musculoskeletal pain, low back pain, Lyme disease accompanied by fibromyalgia syndrome, generalized tendomyopathy, and temporomansibular joint disorder.

15. A method of preventing and/or treating diseases accompanied by chronic musculoskeletal pain which comprises administering an effective amount of a compound having an adenosine A_{2A} receptor antagonistic action or a pharmaceutically acceptable salt thereof.

16. Use of a compound having an adenosine A_{2A} receptor antagonistic action or a pharmaceutically acceptable salt thereof for the manufacture of a prophylactic and/or therapeutic agent for diseases accompanied by chronic musculoskeletal pain.
